# FASCICULE DE BREVET EUROPEEN

(11) **EP 3 448 335 B1**
(45) Date de publication et mention de la délivrance du brevet: **30.10.2024**
(21) Numéro de dépôt: 17725311.9
(22) Date de dépôt: 25.04.2017
(51) Int. Cl.: A61F 7/02, A61F 7/10, A61F 7/00

(54) **ARTICLE COMPRENANT UN MATÉRIAU À FONCTION REFROIDISSANTE**
ARTIKEL MIT EINEM MATERIAL MIT KÜHLFUNKTION
ARTICLE COMPRISING A MATERIAL WITH A COOLING FUNCTION

(30) Priorité: 25.04.2016 FR 1653649
(43) Date de publication de la demande: 06.03.2019
(73) Titulaire: Antartic-Medical, 36400 La Châtre (FR)
(72) Inventeur: PLANCHON, Hubert, 18190 Châteauneuf-sur-Cher (FR); PLANCHON, Olivier, 34990 Juvignac (FR)
(74) Mandataire: Icosa
(86) Numéro de dépôt international: PCT/FR2017/050983
(87) Numéro de publication internationale: WO 2017/187084

(56) Documents cités:
- JP-A- H06 178 792
- US-A1- 2002 042 641
- US-A1- 2006 178 717
- US-A1- 2008 027 523

## Description

### DOMAINE DE L'INVENTION

La présente invention concerne le domaine des articles de type compresse thermique. La présente invention concerne en particulier le domaine de la cryothérapie, et en particulier celui des articles de type compresse thermique réfrigérés, surgelés ou congelés.

### ÉTAT DE LA TECHNIQUE

Le froid est bien connu pour être efficace dans le soin de certaines affections physiques. Le froid est utile pour soulager la douleur ou pour provoquer le dégonflement d'oedèmes. Dans certains cas, il est nécessaire que le froid soit appliqué sur la partie du corps à traiter en respectant des conditions de stérilité. C'est le cas par exemple en présence de plaies ou d'irritations prononcées de la peau susceptibles de s'infecter. Il est par ailleurs connu que l'action antalgique du froid est optimale quand la peau est portée à une température comprise entre +5 et +12°C.

Les compresses réfrigérantes sont souvent des poches qui sont activées par remplissage d'eau puis réfrigération ou congélation.

La demande de brevet américain US2008/0027523 décrit une couverture thermique pour refroidir le corps d'un sujet comprenant un élément de refroidissement comprenant un fluide de refroidissement refroidi au-dessous du point de congélation, cet élément étant destiné à être placé sur différentes parties du corps du sujet. Afin d'obtenir une couverture de refroidissement souple, plusieurs éléments de refroidissement sont avantageusement disposés sur un support souple. Il peut comprendre une couche de renforcement formée, par exemple, par un tissu. Cependant, les éléments de refroidissement ne sont pas détachables les uns des autres pour ajuster les dimensions de la couverture de refroidissement aux dimensions ou à la morphologie du sujet. Ainsi, cette couverture de l'art antérieur n'est pas adaptable en longueur et en largeur.

La demande de brevet européen EP691111 décrit une compresse thermique comprenant un tampon pour une application sur un membre ou le corps d'un animal ou d'un humain, comprenant un réseau d'au moins deux poches étanches interconnectées faites en feuilles de matière plastique souple, chacune des poches contenant un matériau synthétique structuré qui est imprégné par un liquide ayant un point de solidification inférieur à 0°C. Cette compresse comprend un réseau de poches étanches de formes rectangulaires liées par des soudures. Toutefois la flexibilité de cette compresse est limitée : une telle compresse, une fois les poches imprégnées par le liquide et congelées est finalement peu flexible et ne permet pas d'épouser les courbures des zones sur lesquelles elle doit être appliquée.

Il est enfin connu du document JP H06 178792 un dispositif composé de deux feuillets superposés définissant une pluralité de compartiments comprenant une face polymère étanche et une face perméable. Cependant, l'utilisation de polymère étanche de type plastique pour un contact prolongé ne permet pas d'assurer un refroidissement optimal, ni d'assurer la respiration de la peau au niveau de la zone à refroidir.

Il est également connu du brevet EP1607074 un article comprenant au moins un sachet extérieur, étanche à l'eau, comprenant d'une part une compresse à effet refroidissant et d'autre part une poche d'eau, la compresse et la poche d'eau étant au contact l'une de l'autre. Ladite compresse est constituée d'une enveloppe, au moins en partie perméable à l'eau, contenant des particules d'un polymère à forte capacité d'absorption d'eau à l'état sec. Ladite poche d'eau est fermée et supposée être étanche à l'eau, mais est en contact direct avec l'enveloppe contenant le polymère ; or, le matériau constituant la poche d'eau étant notoirement poreux, l'humidité qui s'en échappe provoque une dégradation prématurée du polymère. De ce fait, l'étanchéité de la poche d'eau est limitée dans le temps. La poche d'eau comprend une zone frangible dont la rupture permet de transférer l'eau vers l'enveloppe comprenant le polymère. La compresse peut consister en une suite de sachets alignés selon une seule dimension de l'espace. Une telle compresse, une fois les poches imprégnées par le liquide et congelées, présente les mêmes inconvénients que les autres compresses de l'art antérieur : elle est finalement peu flexible. Lorsqu'elle l'est, sa flexibilité est limitée uniquement suivant une seule dimension de l'espace. Ainsi, cette compresse ne convient qu'aux parties du corps sensiblement plates ou cylindriques, telles que l'abdomen, le bras ou la cuisse. En revanche, elle ne peut pas épouser les courbures des zones du corps aux formes complexes, telles que notamment la main, le genou la cheville, la mâchoire, le crâne, etc.

Ainsi, les compresses de l'art antérieur ne sont pas adaptables en longueur et en largeur.

Il est également connu du brevet américain US2006/0178717 une couverture thermique configurable, en tissu ou en film déchirable qui évite de précommander une taille pour un patient. La couverture comprend une pluralité de compartiments scellés discrets. Chaque compartiment est complètement circonscrit par un joint continu qui comprend une structure de séparation pour permettre le détachement d'un ou plusieurs compartiments. Toutefois, le risque de déchirer le tissu, et donc la compresse, de manière involontaire lorsque l'on cherche à séparer un compartiment, n'est pas géré. Ainsi, cette couverture amovible de l'art antérieur est susceptible de se déchirer lors du détachement d'un ou plusieurs compartiments.

Finalement, un dernier besoin n'est pas couvert par les dispositifs antérieurs en général et, en particulier, ceux suscités : il s'agit du confort au froid. En effet, si l'application de froid mène, dans des conditions optimales, à l'anesthésie de la peau de la zone traitée, il reste que la sensation de froid avant l'obtention de cet effet peut prendre la forme d'une sensation de brûlure insupportable, ce qui réduit considérablement l'acceptabilité de tels dispositifs. La sensation de brûlure est notoirement plus prononcée lorsque le froid est appliqué sur une articulation, alors que les articulations sont des cibles privilégiées de la cryothérapie.

Il existe donc de multiples besoins dans l'utilisation d'articles contenant des matériaux à fonction thermique refroidissante, auxquels les dispositifs actuels ne répondent pas. En particulier, il existe un besoin pour un article qui soit en mesure :
- de s'adapter en taille (longueur, largeur et épaisseur) et en forme,
- dans certains cas, de pouvoir être conditionné en grandes longueurs, par exemple de plusieurs mètres, et utilisée progressivement selon les besoins,
- de garantir le non-déchirement lors du détachement d'un compartiment de l'article,
- de délivrer le froid dans des conditions de confort optimales pour le patient.

### DÉFINITIONS

Dans la présente invention, les termes ci-dessous sont définis de la manière suivante :
- « **Adhésif** » : matériau qui permet d'assembler et de retenir ensemble deux objets, de manière réversible ou irréversible,
- « **Compartiment** » : volume enfermé dans les trois directions de l'espace, de telle sorte que ce volume ne communique ni avec l'extérieur, ni avec d'autres compartiments adjacents, à moins qu'une paroi ou une jonction n'ait été ouverte ou rompue,
- « **Compresse** » : article de type compresse thermique a effet réfrigérant ou chauffant,
- « **Conduction** » : mode de transport de calories dans un matériau, généralement solide, par propagation de proche en proche de l'agitation moléculaire,
- « **Convection** » : mode de transport de calories dans un fluide par mouvement du fluide lui-même,
- « **Découpe** » : coupe séparant totalement deux parties d'un élément,
- « **Emballage** » : conditionnement destiné à contenir et à protéger des marchandises, notamment à éviter leur contamination, à permettre leur manutention et leur acheminement du producteur au consommateur ou à l'utilisateur, et optionnellement à assurer leur présentation,
- « **Environ** » : le terme « **environ** », placé devant un nombre, signifie plus ou moins 10% de la valeur nominale de ce nombre,
- « **Feuillets** » : enveloppe supérieure et, respectivement, inférieure des compartiments. Les deux feuillets sont soudés entre eux le long de lignes qui délimitent lesdits compartiments. Un feuillet peut être éventuellement constitué de plusieurs épaisseurs superposées,
- « **Couche de renforcement** » : désigne un tissu ou un film superposé à un article pour en garantir l'intégrité,
- « **Milieu aqueux** » : solution aqueuse, phase liquide, comprenant essentiellement de l'eau, uniquement de l'eau, ou un mélange d'eau et d'additifs dissous ; dans un mode de réalisation, le milieu aqueux peut contenir un agent actif tel que décrit dans la présente invention ; dans un mode de réalisation, le milieu aqueux peut contenir des arômes ou fragrances, des colorants, des actifs qui apportent une sensation de froid ; dans un mode de réalisation, le milieu aqueux est une solution saline du type sérum physiologique,
- « **Non-tissé** » : produit manufacturé, constitué d'un voile, d'une nappe ou d'un matelas de fibres réparties directionnellement ou aléatoirement, et dont la cohésion interne est assurée par des méthodes mécaniques, physiques et/ou chimiques et/ou par combinaison de ces divers procédés, à l'exclusion du tissage et du tricotage,
- « **Pavage** » : partition du plan telle que chaque point du plan appartient à une tuile et à une seule. En d'autres termes, un pavage comprend des tuiles qui sont juxtaposées sans se superposer ni laisser d'espace entre elles. Dans un autre mode de réalisation, les tuiles peuvent être identiques entre elles, dans le sens où chaque tuile peut se déduire de toute autre tuile par translation symétrie, rotation et/ou homothétie. Dans un autre mode de réalisation, les tuiles peuvent être de forme et/ou de taille différente dans un même pavage. Une tuile peut avoir une forme quelconque, notamment une tuile peut être notamment carrée, rectangulaire, triangulaire, hexagonale, circulaire. Une tuile peut être pleine ou évidée. De par sa définition, un pavage est infini. Dans la présente invention, on dira qu'un article de superficie finie « est un pavage » quand il est en réalité l'intersection entre un pavage stricto-sensu et le périmètre dudit objet,
- « **Prédécoupe** » : une zone de l'espace entre deux tuiles adjacentes, sur tout ou partie du périmètre d'une tuile où les deux feuillets sont joints mais présentent une découpe partielle, généralement sous forme de pointillés, permettant de séparer deux tuiles,
- « **Matériau absorbant** » : matériaux ou poudre pouvant absorber et conserver de très grandes quantités d'un liquide en comparaison avec sa propre masse. Selon un mode de réalisation, un matériau absorbant placé dans une tuile peut absorber plusieurs fois sa masse en liquide et occuper ainsi un volume. Le liquide absorbé peut être un milieu aqueux ou un liquide organique,
- « **Solidaire** » : qualifie l'état de deux éléments distincts d'un même ensemble qui n'ont pas de mouvement relatif l'un par rapport à l'autre et qui sont physiquement liés,
- « **Soudure de mélange** » : assemblage de matériaux qui conduit à leur mélange partiel et qui a pour objet d'assurer la continuité de la matière à assembler. Le terme de soudure de mélange désigne donc ici la soudure dans l'acception la plus classique du terme. Souder consiste à assembler des matériaux grâce à un procédé de chauffage qui provoque la fusion partielle des parties à assembler,
- « **Soudure d'affaiblissement** » : soudure de matériaux par mélange des matériaux et réduction de l'épaisseur de la soudure ; la réduction de l'épaisseur de la soudure a pour effet d'affaiblir ou de fragiliser la soudure, qui devient alors frangible,
- « **Soudure augmentée** » : zone de soudure le long de laquelle le trait de soudure augmente en largeur,
- « **Point de jonction** » : zone de connexion renforcée entre deux tuiles, d'une largeur comprise entre 0,1 et 2 mm, où la soudure d'affaiblissement est interrompue,
- « **Ligne frangible** » : alternance de soudures d'affaiblissement et de points de jonction définissant une zone par laquelle deux tuiles peuvent être séparées entre elles,
- « **À proximité** » : par rapport à un point d'une tuile situé sur une longueur ou une largeur d'une tuile, signifie une distance de 1 à 10%, de préférence 2 à 5% de la longueur ou de la largeur de la tuile, par rapport à ce point.

### SOMMAIRE DE L'INVENTION

L'invention est définie par la revendication indépendante annexée. D'autres modes de réalisation de l'invention sont illustrés dans les revendications dépendantes.

### DESCRIPTION

La présente description a pour objet de fournir un article qui évite les inconvénients des dispositifs de l'art antérieur, et qui réponde aux besoins évoqués ci-dessus.

Ainsi, un exemple concerne, dans un premier aspect, un article composé de deux feuillets superposés et soudés pour définir un pavage comprenant au moins trois, de préférence au moins quatre tuiles ; chaque tuile comprend au moins trois côtés et trois sommets ; ces tuiles se déploient dans au moins deux directions de l'espace définissant un plan ; les tuiles sont adjacentes et connectées les unes aux autres par au moins un de leurs côtés ; la solidarisation du périmètre de chaque tuile définit un compartiment intérieur, qui est un espace entre les deux feuillets, incluant au moins un matériau absorbant de milieu aqueux ou un matériau de remplissage ; les tuiles sont fermées et connectées les unes aux autres par au moins une soudure ; la soudure est réalisée par ultrasons, thermiquement ou par tout type de soudure connu de l'homme du métier ; la soudure est composée d'une ligne frangible constituée d'une succession de soudures d'affaiblissement et de points de jonction ou d'une prédécoupe bordée de part et d'autre d'une soudure de mélange ; la soudure de mélange étant élargie à proximité du sommet de chaque tuile de telle sorte que le coin des tuiles est renforcé par une surface de soudure augmentée ; de préférence, la soudure de mélange a une largeur à proximité du sommet de chaque tuile d'au moins 10% supérieure à la largeur de la soudure de mélange des côtés des tuiles ; très préférentiellement, la soudure de mélange forme un triangle de 1 à 10, de préférence 2 à 8, très préférentiellement 3 à 5 mm de côté à chaque sommet de la tuile.

Cette soudure élargie au niveau des sommets des tuiles permet de diminuer les forces qui s'exercent sur l'angle central lorsque le compartiment est gonflé (soit par le matériau de remplissage, soit par le matériau absorbant qui a été mis en contact avec un milieu aqueux) et d'éviter par là des déchirures, notamment lors de la séparation des tuiles. Par ailleurs, la ligne frangible est telle qu'il est possible de séparer les tuiles manuellement en exerçant deux forces de sens opposés et de direction perpendiculaire au plan formé par les tuiles. Cette soudure est telle, en revanche, qu'il n'est pas possible de séparer les tuiles manuellement en exerçant deux forces de sens opposés et de direction parallèle au plan formé par les tuiles.

Le présent exemple a donc pour effet de procurer une séparation très simple de certaines tuiles de l'article, tout en assurant l'intégrité des tuiles restantes sur l'article.

Ainsi, dans un mode de réalisation, les tuiles sont détachables les unes des autres (avant ou après l'activation et/ou la congélation) sans effectuer de déchirure, sans utiliser d'outillage particulier et sans affaiblir la structure de pavage.

Dans un mode de réalisation, la soudure d'affaiblissement entre deux tuiles est continue au sommet de chaque tuile.

Dans un mode de réalisation, la soudure d'affaiblissement entre deux tuiles est discontinue en environ trois points par centimètre, de manière à générer au moins trois points de jonction.

Dans un mode de réalisation, la soudure d'affaiblissement entre deux tuiles est discontinue en au moins deux points par centimètre, de manière à générer au moins deux points de jonction de soudure de mélange.

Dans un mode de réalisation, la ligne frangible est située sur le périmètre de la tuile, plus exactement sur tout ou partie du périmètre de la tuile.

Dans un mode de réalisation, la ligne frangible se situe sur tout le périmètre de la tuile, ce qui permet de détacher ladite tuile du pavage dans lequel elle se trouve.

Dans un mode de réalisation, la soudure d'affaiblissement est située au bord extérieur d'une soudure de mélange. Dans un mode de réalisation, la tuile comprend d'autres soudures d'affaiblissement que la soudure d'affaiblissement de connexion à une ou plusieurs tuiles adjacentes.

Dans un mode de réalisation, au moins un des feuillets est constitué d'une double épaisseur ; de préférence, chacun des deux feuillets est doublé. Cette doublure présente quatre avantages majeurs. Tout d'abord, elle diminue le givre lors de la congélation. Ensuite, elle augmente considérablement le confort au froid. De plus, elle retarde la migration du gel contenu dans le compartiment. Enfin, augmente la solidité de l'article, ce qui permet de le réutiliser plusieurs fois.

Dans un mode de réalisation, l'article ne comprend pas une couche de renforcement.

Dans un premier mode de réalisation, l'article est non stérile.

Dans un second mode de réalisation, l'article est stérile. De préférence, il est stérilisé par rayons gamma ou suivant les procédés de stérilisation connus de l'homme du métier.

Dans un troisième mode de réalisation, l'article est décontaminé par rayons gamma.

Dans un mode de réalisation, la surface du compartiment occupe 10 à 99%, de préférence 50 à 95%, très préférentiellement 70 à 90% de la surface de la tuile.

Dans un premier mode de réalisation, la tuile a une forme identique à celle du ou des compartiments qu'elle délimite.

Dans un second mode de réalisation, la tuile a une forme différente de celle du ou des compartiments qu'elle délimite.

Dans un mode de réalisation, le matériau absorbant de milieu aqueux comprend ou est constitué d'une chaîne de poly(acide acrylique) ou de ses sels. Dans un mode de réalisation, le matériau absorbant de milieu aqueux est constitué d'une seule nature de polymère.

Dans un mode de réalisation, le matériau absorbant de milieu aqueux ne comprend pas ou n'est pas constitué d'une chaîne de poly(acide acrylique) ou de ses sels.

Dans un mode de réalisation, le matériau absorbant de milieu aqueux comprend des particules homogènes et ne comprend pas de particule en deux parties de type coeur et coquille.

Dans un mode de réalisation, le matériau absorbe 0,1 à 20% de la capacité d'eau qu'il pourrait absorber à l'air libre.

Selon un mode de réalisation, au moins une des faces externes des feuillets est revêtue, en tout ou partie, d'un adhésif.

Selon un mode de réalisation, au moins une des faces externes des feuillets est revêtue, en tout ou partie, d'un matériau qui adhère sans coller à la peau, par exemple de type silicone, en particulier un gel silicone.

Selon un mode de réalisation, au moins un des feuillets est perméable à un liquide d'activation, qui est de préférence de l'eau ou un milieu aqueux.

Selon un mode de réalisation, au moins un feuillet, et de préférence tous les feuillets, sont en textile non-tissé.

Selon un mode de réalisation, les feuillets ne comprennent ni couche isolante ni matériau isolant.

Dans un mode de réalisation, les tuiles ne sont pas thermoformées.

Dans un mode de réalisation particulier, au moins un des deux feuillets est thermoformé pour former, avec l'autre feuillet superposé, des compartiments en trois dimensions, notamment parallélépipédique, polyédrique, sphérique ou hémisphérique. Dans un mode de réalisation, l'article est entouré d'une lisière, qui est un bord longitudinal. La lisière peut être réalisée par solidarisation des deux feuillets sur tout ou partie des bords de l'article.

Dans un mode de réalisation, l'article est dans un emballage qui comprend au moins un film microporeux ou microperforé perméable à l'eau mais barrière aux microbes, bactéries et/ou virus.

Dans un autre mode de réalisation, l'emballage est multipoches et comprend une première poche comprenant l'article, une seconde poche, indépendante de la première poche et étanche par rapport à la première poche, comprenant ou apte à recevoir un milieu aqueux Les deux poches sont solidaires par un de leurs côtés et comprennent un moyen de mise en communication fluidique de la seconde poche vers la première poche.

Dans tous les modes de réalisation comprenant un emballage, l'emballage comprend au moins une soudure pelable ou déchirable, et de préférence au moins deux soudures pelables adjacentes, pour une extraction facile de l'article.

Ainsi, un exemple concerne un article qui peut être rempli d'un matériau absorbant de milieu aqueux et qui est activable par mise au contact d'un milieu aqueux, ladite activation ayant pour effet de gonfler un mélange d'eau et de matériau absorbant qui se trouve dans le compartiment intérieur de la tuile. L'activation est suivie d'une étape de refroidissement, congélation ou surgélation. Alternativement, l'article selon l'invention est rempli et gonflé d'un matériau de remplissage et nécessite uniquement d'être refroidi, congelé ou surgelé pour former une compresse thermique.

Dans un second aspect, un exemple concerne donc une compresse thermique comprenant un article selon l'invention, qui a été activé si nécessaire (si le matériau la contenant est un matériau absorbant de milieu aqueux, l'activation se fait par mise en contact de l'article selon l'invention avec un milieu aqueux) et refroidie, congelée ou surgelée. Dans un mode de réalisation, la compresse thermique selon l'invention a pour fonction de communiquer du froid à tout objet ou corps à son contact, par transport de la chaleur latente de fusion absorbée lors de la transition de la phase solide à la phase liquide. Dans un mode de réalisation, la compresse thermique selon l'invention a pour fonction de communiquer du froid à tout objet ou corps à son contact, par convection ou par conduction.

Dans un troisième aspect, un exemple concerne un rouleau constitué par l'enroulement sur lui-même de l'article selon l'invention.

Dans un quatrième aspect, un exemple concerne un casque comprenant un article selon l'invention. De préférence, ce casque comprend ou est constitué d'une charlotte, de préférence jetable, et d'au moins un article selon l'invention. Avantageusement, ce casque est un casque de chimiothérapie destiné à lutter contre la chute des cheveux liée à un traitement de chimiothérapie.

Dans un cinquième aspect, un exemple concerne un procédé de fabrication de l'article selon l'invention comprenant un défilement continu d'au moins deux feuillets non soudés devant deux stations de soudures : une longitudinale, une autre transversale. La station de soudure longitudinale comprend au moins 3 zones de soudure à savoir une première zone de bordure, au moins une zone intermédiaire et une seconde zone de bordure, la soudure longitudinale étant une soudure d'affaiblissement bordée de deux soudures de mélange. La station de soudure transversale comprend au moins trois zones de soudure, à savoir une première zone de bordure, au moins une zone intermédiaire et une seconde zone de bordure, ladite soudure transversale étant une soudure d'affaiblissement bordée de deux soudures de mélange.

La distance entre deux zones de soudure longitudinale définit la longueur d'une tuile et la distance entre deux zones transversales définit la largeur d'une tuile.

Le procédé de fabrication comprend en outre le dépôt d'un matériau absorbant de milieu aqueux dans chaque tuile préalablement à sa soudure complète.

Dans un sixième aspect, un exemple concerne un dispositif de mise en oeuvre du procédé selon l'invention. Le dispositif comprend une station de soudure longitudinale comprenant au moins trois moyens de soudure, chaque moyen comprenant 2 rondelles de soudure de mélange entourant une rondelle de soudure d'affaiblissement pouvant être à encoches, de façon à réaliser une soudure d'affaiblissement discontinue générant des points de jonction de soudure de mélange, ou bien sans encoche, de façon à réaliser une soudure d'affaiblissement continue. Le dispositif comprend par ailleurs une station de soudure transversale comprenant 2 rondelles de soudure de mélange entourant une rondelle de soudure d'affaiblissement pouvant être à encoches, de façon à réaliser une soudure d'affaiblissement discontinue, ou bien sans encoche, de façon à réaliser une soudure d'affaiblissement continue.

### BRÈVE DESCRIPTION DES FIGURES

Les **Figures 1A**, **2A**, **3A**, **4A** et **5A** sont des vues de face montrant plusieurs modes de réalisation d'une tuile.
Les **Figures 1B**, **2B**, **3B**, **4B** et **5B** sont des vues de face montrant plusieurs modes de réalisation de l'article selon l'invention.
La **Figure 6** est une coupe d'un mode de réalisation de l'article selon l'invention.
La **Figure 7A** est une vue de face d'un article thermoformé selon l'invention.
La **Figure 7B** est une vue de côté d'un article thermoformé selon l'invention.
La **Figure 8** montre un article selon l'invention inclut dans un emballage multipoches.
La **Figure 9A** et **9B** montrent un article selon l'invention inclut dans un emballage monopoche.
La **Figure 10** représente l'utilisation d'un article selon l'invention, en application cutanée sur un genou.
La **Figure 11** représente un dispositif de fabrication d'un mode de réalisation de l'article selon l'invention.

### RÉFÉRENCES

1 - Article,
2 - Tuile,
3 - Compartiment,
4 - Feuillet,
4A - Face externe du feuillet,
4B - Face interne du feuillet,
5 - Soudure de mélange,
6 - Soudure d'affaiblissement,
7 - Point de jonction,
8 - Ligne frangible,
9 - Dispositif de fabrication,
91 - Pièces mécaniques pour le guidage et la superposition des feuillets,
92 - Module de solidarisation verticale,
923 - Module de solidarisation horizontale,
921, 931 - Ensemble ultrasons,
922, 932 - Tambour de solidarisation,
94 - Tapis de transfert,
941 - Bande transporteuse,
942 - Moteur,
95 - Unité de distribution,
951 - Sac,
952 - Came de transfert,
953 - Réservoir intermédiaire,
954 - Doseurs mécaniques,
955 - Couloir mécanique,
10 - Emballage multipoches,
101 - Première poche,
102 - Deuxième poche,
11 - Emballage monopoche,
12 - Moyen de mise en communication fluidique,
13 - Soudure pelable ou déchirable.

### DESCRIPTION DÉTAILLÉE

La présente invention se comprendra mieux à la lecture de la description des figures qui illustrent non-limitativement l'invention.

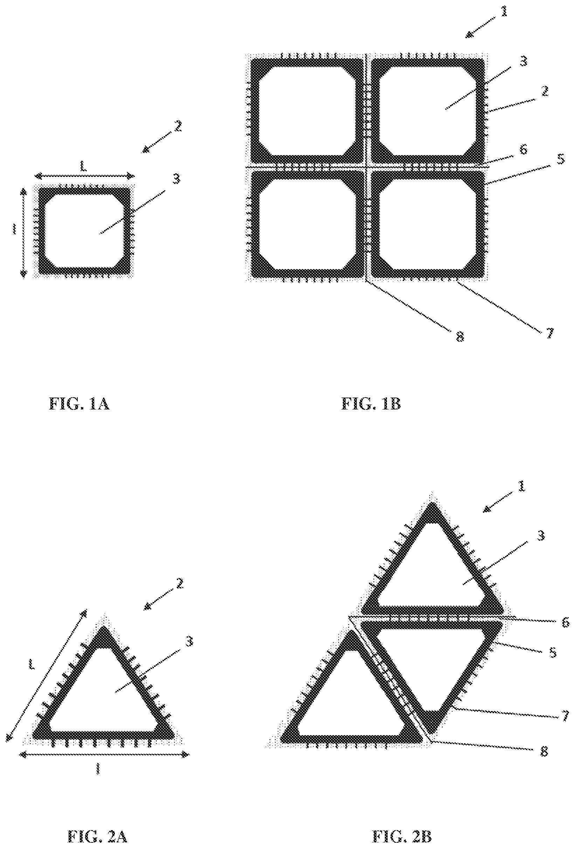

Les Figures 1A, 2A, 3A, 4A et 5A représentent différents modes de réalisation d'une tuile **2** en vue de face. Chaque tuile **2** comprend un compartiment **3.**

En particulier la Figure 1A montre une tuile **2** de forme carrée comprenant un compartiment **3** de forme carré ; la tuile **2** est de dimensions légèrement supérieures à celle du compartiment **3.**

La Figure 2A montre une tuile **2** de forme triangulaire comprenant un compartiment **3** de forme triangulaire ; la tuile **2** est de dimensions légèrement supérieures à celles du compartiment **3.**

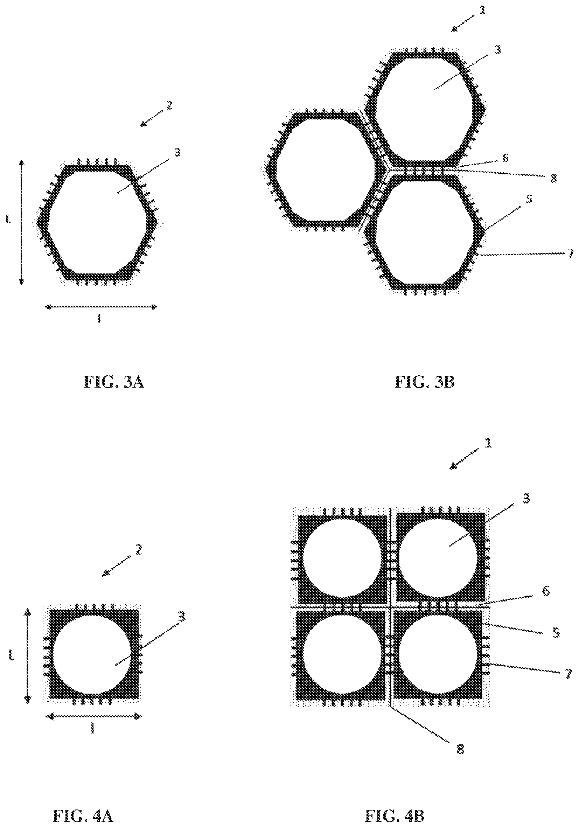

La Figure 3A montre une tuile **2** de forme hexagonale comprenant un compartiment **3** de forme hexagonale ; la tuile **2** est de dimensions légèrement supérieures à celles du compartiment **3.**

La Figure 4A montre une tuile **2** de forme carrée comprenant un compartiment **3** de forme circulaire ; la tuile **2** est de dimensions légèrement supérieures à celles du compartiment **3.**

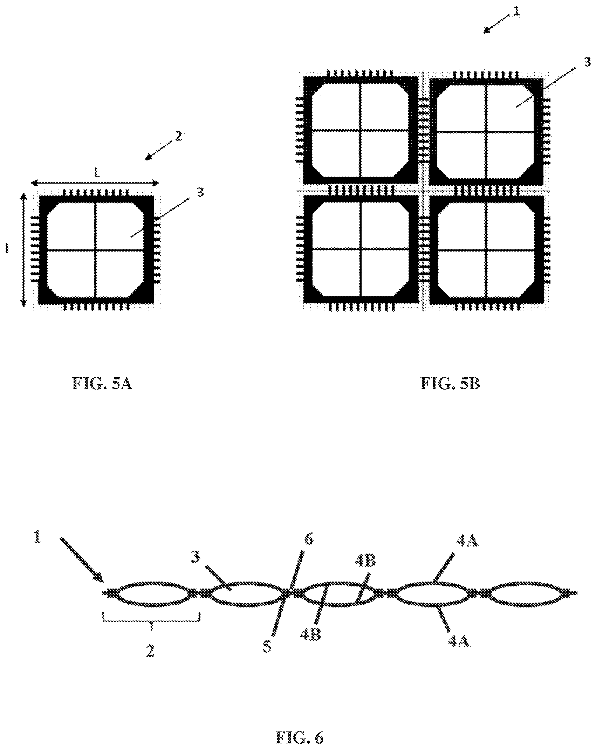

La Figure 5A montre une tuile **2** de forme carrée comprenant quatre compartiments **3** de forme carrée ; la tuile **2** est de dimensions légèrement supérieures à celles des quatre compartiments **3.**

Les Figures 1B, 2B, 3B, 4B et 5B représentent différents modes de réalisation d'un article **1** selon l' invention vue de face comprenant une pluralité de tuiles **2** telles que représentées respectivement aux Figures 1A, 2A, 3A, 4A et 5A.

L'article **1** comprend au moins trois tuiles **2.** Cette propriété lui assure la fonctionnalité, recherchée par l'invention, d'être flexible dans toutes les directions.

L'article **1** comprend deux feuillets **4** qui ont chacun une face externe **4A** et une face interne **4B.**

Comme illustré sur la Figure 6, chaque tuile **2** comprend au moins un compartiment **3,** délimité par deux soudures de mélange **5** qui bordent une soudure d'affaiblissement **6.** Ces soudures solidarisent les faces internes **4B** des feuillets. Les tuiles **2** sont connectées les unes des autres par soudure d'affaiblissement le long de leur périmètre ; cette soudure d'affaiblissement rend les tuiles **2** séparables à mains nues, sans qu'il soit besoin d'un quelconque outil.

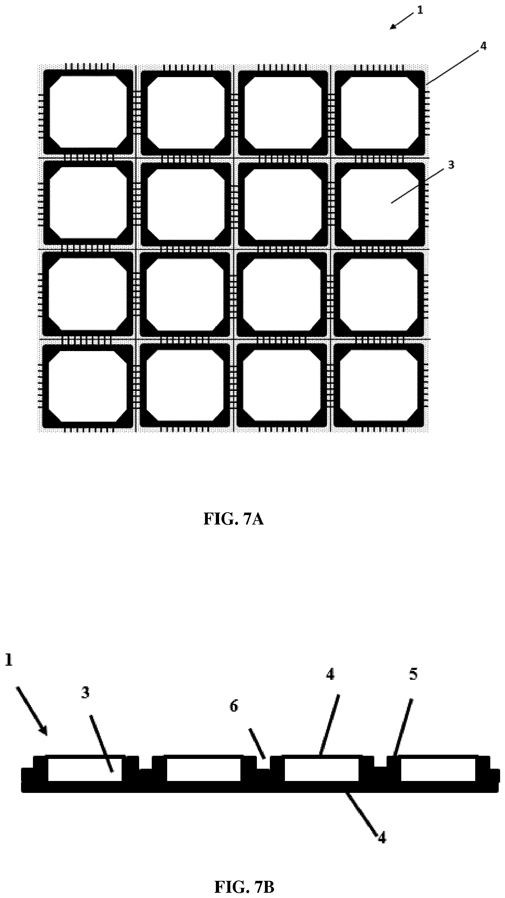

Les Figures 7A et 7B ont pour objet de montrer respectivement une vue de face et une vue de côté d'un mode de réalisation particulier de l'invention, dans lequel au moins un des deux feuillets **4** est thermoformé pour former, avec l'autre feuillet superposé **4**, des compartiments **3** en trois dimensions, notamment parallélépipédique, polyédrique, sphérique ou hémisphérique. En particulier, sur la Figure 7B, le feuillet supérieur **4** est thermoformé et le feuillet inférieur **4** n'est pas thermoformé.

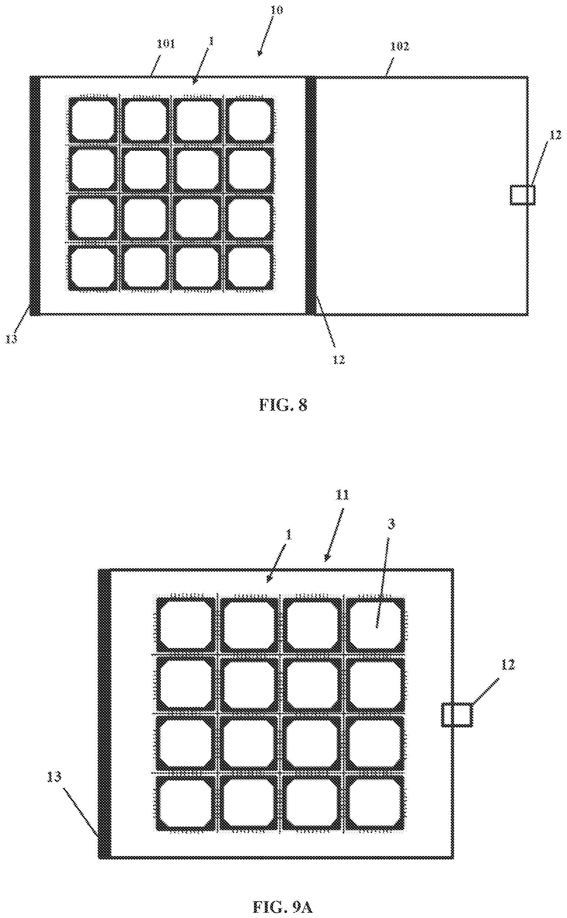

La Figure 8 montre un article **1** selon l'invention, qui est inclus dans un emballage multipoches **10.** Avantageusement, l'emballage multipoches **10** comprend :
- une première poche **101** comprenant un article **1** selon l'invention,
- une seconde poche **102**, comprenant (ou apte à comprendre) un liquide d'activation stérile ou non, du matériau à fonction thermique,
- les deux poches **101**, **102** sont juxtaposées par un de leurs côtés et de préférence solidaires, sur ledit côté ; la poche **101** présente une soudure pelable ou déchirable **13** permettant d'extraire l'article ; dans un mode de réalisation, la poche **101** comprend au moins deux soudures **13** adjacentes pelables,
- les deux poches comprenant un moyen de mise en communication fluidique **12**, qui est de préférence une soudure pelable, entre la seconde poche **102** et la première poche **101.**

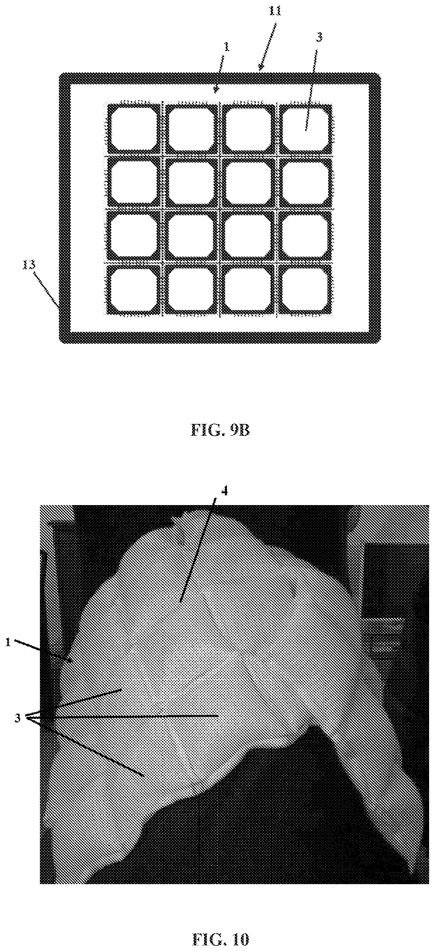

Les Figures 9A et 9B illustrent un article **1** selon l'invention, qui est inclus dans un emballage monopoche **11.** Dans la figure 9A est montré un article **1** maintenu stérile dans l'emballage **11.** Dans ce mode de réalisation, l'emballage **11** est clos par tout moyen, notamment par thermosoudage de son périmètre, qui isole l'article **1** de l'environnement extérieur afin de conserver sa stérilité. Dans un mode de réalisation, la soudure **13** est pelable. Dans un mode de réalisation, l'emballage **11** comprend au moins deux soudures adjacentes pelables. Dans un mode de réalisation, le liquide d'activation de l'article **1** est un milieu aqueux stérile, qui est ajouté au moyen d'une seringue préremplie ou d'une poche préremplie par l'intermédiaire d'un moyen de mise en communication fluidique **12 ;** ainsi, l'ensemble du contenu de l'emballage monopoche peut être conservé en conditions de stérilité. Dans le mode de réalisation de la figure 9B, l'emballage comprend un feuillet microporeux ou microperforé (microporosité ou microperforation non représentée), et toutes ses soudures **13** périmétrales sont pelables. Selon un second mode de réalisation, mis en oeuvre dans un cadre non stérile, l'article **1** est retiré de l'emballage monopoche **11** afin de permettre l'immersion de l'article **1** dans un liquide d'activation.

Selon un troisième mode de réalisation, l'emballage monopoche **11** comprend au moins une partie microporeuse ou microperforée, de sorte à être perméable à une solution aqueuse pour permettre l'absorption du liquide d'activation. Dans ce dernier mode de réalisation, l'emballage **11** peut être plongé directement dans un liquide d'activation, et l'article **1** n'être retiré de l'emballage **11** qu'après avoir subi sa transformation thermique.

La Figure 10 représente l'utilisation d'un article **1** selon l'invention, en application cutanée sur un genou. Grâce à la flexibilité de l'article **1**, l'article recouvre le genou et apporte du froid sur toutes les parties ciblées. L'article 1 présenté sur le mode de réalisation de cette Figure a été partiellement séparé dans sa largeur au niveau de la ligne frangible ou de la prédécoupe. Cette séparation permet d'optimiser la surface recouverte en évitant la formation d'un pli et d'augmenter le froid sur une partie du corps sur laquelle la partie séparée de l'article est superposée sur une autre partie de ce même article.

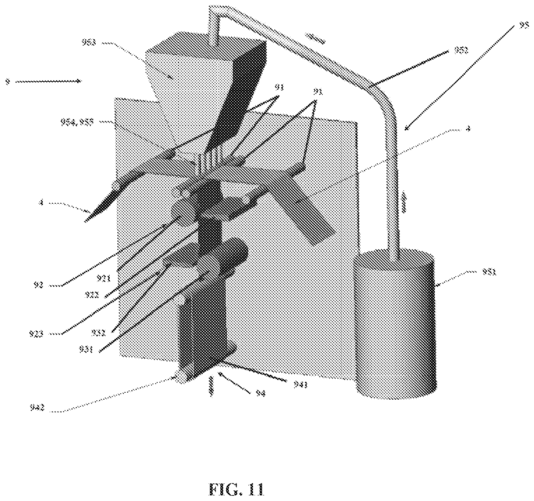

La Figure 11 représente un dispositif de fabrication 9 d'un mode de réalisation de l'article 1 selon l'invention. Ce dispositif comprend :
- deux modules de pré-déroulage (non représenté) des feuillets **4**, en tissé ou en non-tissé, pour amener les feuillets **4** sans tension,
- deux ensembles de pièces mécaniques **91** pour le guidage et la superposition des feuillets **4**, pendant les opérations de solidarisation,
- un module de solidarisation **92** vertical, permettant de réaliser plusieurs compartiments, comprenant :
   - un ensemble ultrasons **921** comprenant une unité de réglage sur rails et patins asservi par un moteur réducteur, une sonotrode de solidarisation acier, un ensemble convertisseur et un booster 20 KHz ultrasons, et
   - un tambour de solidarisation **922** équipé de pièces mécaniques, de molettes de solidarisation pour la fermeture de la poche et de molette de coupe/solidarisation pour la réalisation de la ligne frangible ou de la prédécoupe, ces molettes sont assemblables et positionnables suivant le format de l'article à réaliser et d'un moteur réducteur asynchrone,
- un module de solidarisation **923** horizontal, permettant de réaliser la fermeture des poches composant l'article, comprenant :
   - un ensemble ultrasons **931** comprenant une unité de réglage sur rails et patins asservi par un moteur réducteur, une sonotrode de solidarisation acier, un ensemble convertisseur et un booster 20 KHz ultrasons, et
   - un tambour de solidarisation **932** équipé de pièces mécaniques, de barres de solidarisation longitudinales, et de barres de coupe/solidarisation pour la réalisation de la ligne frangible ou de la prédécoupe de la poche dans le sens longitudinal et d'un moteur réducteur asynchrone,
- un tapis de transfert **94** à dépression permettant la motorisation et le transfert de l'article réalisé comprenant :
   - un ensemble de pièces mécaniques équipé d'une sole ajourée,
   - une bande transporteuse **941** ajourée permettant l'adhérence par dépression, et
   - un moteur réducteur asynchrone **942**,
   - une unité de distribution **95** de la poudre polyacrylate comprenant :
      - un ensemble pneumatique de distribution de la poudre du sac **951** vers le réservoir intermédiaire **953** composé d'un ensemble pneumatique et d'une came de transfert **952**,
      - un réservoir intermédiaire **953** pour le stockage de la poudre en attente de distribution,
      - cinq doseurs mécaniques **954** sélectionnables permettant le dosage volumétrique de la poudre à distribuer. Chaque doseur est composé d'un ensemble de pièces mécaniques et d'un vérin pneumatique pour le fonctionnement du tiroir de sélection. Chaque doseur est sélectionnable en relation avec le format de l'article à fabriquer, et
      - un ensemble de cinq couloirs mécaniques **955** assurant le transfert de la poudre dans la poche présélectionnée et définie.

## Revendications

1. Article (1) thermique refroidissant composé d'au moins deux feuillets (4) superposés et soudés par une soudure de mélange pour définir un pavage comprenant au moins trois tuiles (2), ladite soudure de mélange (5) étant élargie à proximité du sommet de chaque tuile (2) de telle sorte que le coin (2) des tuiles est renforcé par une surface de soudure augmentée, chaque tuile (2) comprenant au moins trois côtés et trois sommets, lesdites tuiles (2) se déployant dans au moins deux directions de l'espace définissant un plan, lesdites tuiles (2) étant adjacentes et connectées les unes aux autres par au moins un de leurs côtés, lesdites tuiles (2) définissant un compartiment (3), qui est un espace entre les deux feuillets (4), le compartiment incluant au moins un matériau configuré pour être refroidi, congelé ou surgelé, choisi parmi un matériau absorbant de milieu aqueux ou un matériau de remplissage, lesdites tuiles (2) étant fermées et connectées les unes aux autres par au moins une soudure, ***caractérisé en ce que*** la soudure est composée d'une ligne frangible (8) constituée d'une succession régulière de soudures d'affaiblissement (6) et de points de jonction (7), la soudure d'affaiblissement (6) entre deux tuiles (2) étant continue au niveau du sommet de chaque tuile (2).

2. Article (1) thermique refroidissant selon la revendication **1**, dans lequel au moins un des feuillets est perméable à un milieu aqueux et le matériau est un matériau absorbant de milieu aqueux qui est activable par mise au contact avec le milieu aqueux, ladite activation ayant pour effet de gonfler un mélange d'eau et de matériau absorbant au sein du compartiment.

3. Article (1) thermique refroidissant selon la revendication **1** ou **2**, dans lequel le matériau absorbant de milieu aqueux est de la poudre de polyacrylate.

4. Article (1) thermique refroidissant selon l'une des revendications **1** à **3**, dans lequel au moins un des feuillets (4) et, de préférence, chacun des deux feuillets (4) est constitué d'une superposition de plusieurs épaisseurs de matériau.

5. Article (1) thermique refroidissant selon l'une quelconque des revendications **1** à **4**, dans lequel la soudure d'affaiblissement (6) entre deux tuiles (2) est discontinue en au moins deux points par centimètre, de manière à générer au moins deux points de jonction (7) de soudure de mélange (5).

6. Article (1) thermique refroidissant selon l'une quelconque des revendications **1** à **5**, dans lequel la surface du compartiment (3) occupe 10 à 99%, de préférence 50 à 95%, très préférentiellement 70 à 90% de la surface de la tuile (2).

7. Article (1) thermique refroidissant selon l'une quelconque des revendications **1** à **6**, dans lequel au moins une des faces externes des feuillets (4) est revêtue, en tout ou partie, d'un adhésif.

8. Système comprenant un emballage et un article (1) thermique refroidissant selon l'une quelconque des revendications **1** à **7**, dans lequel ledit article (1) thermique refroidissant est dans ledit emballage qui comprend au moins un film microporeux ou microperforé perméable à l'eau mais barrière aux microbes, bactéries et/ou virus.

9. Dispositif comprenant un emballage multipoche (10) et un article (1) thermique refroidissant selon l'une quelconque des revendications **1** à **7**, ledit emballage multipoche (10) comprenant :
- une première poche (101) comprenant ledit article (1) thermique refroidissant,
- une seconde poche (102), indépendante de la première poche et étanche par rapport à la première poche, comprenant ou apte à recevoir un milieu aqueux,
- les deux poches étant solidaires par un de leurs côtés, et
- les deux poches comprenant un moyen de mise en communication fluidique (12) de la seconde poche vers la première poche.

10. Compresse thermique refroidissante comprenant au moins un article (1) thermique refroidissant selon l'une quelconque des revendications **1** à **7**, dans lequel le matériau est un matériau absorbant de milieu aqueux qui a été activé par mise en contact avec un milieu aqueux, et qui a été refroidi, congelé ou surgelé.

11. Compresse thermique refroidissante comprenant au moins un article (1) thermique refroidissant selon l'une quelconque des revendications **1** à **7**, dans lequel le matériau est un matériau de remplissage qui a été refroidi, congelé ou surgelé.

12. Rouleau comprenant au moins un article (1) thermique refroidissant selon l'une quelconque des revendications **1** à **7** ou au moins un système selon la revendication **8** enroulé sur lui-même.

13. Casque, de préférence casque de chimiothérapie pour lutter contre la chute des cheveux liée à un traitement de chimiothérapie, comprenant une charlotte, de préférence jetable, et au moins un article (1) thermique refroidissant selon l'une quelconque des revendications **1** à **7**, au moins un système selon la revendication **8** ou au moins un dispositif selon la revendication **9.**

14. Procédé de fabrication en continu d'un article (1) thermique refroidissant selon l'une quelconque des revendications **1** à **7**, d'un système selon la revendication **8** ou d'un dispositif selon la revendication **9** comprenant un défilement continu d'au moins deux feuillets (4) non soudés devant deux stations de soudures : une longitudinale, une autre transversale ; la station de soudure longitudinale comprenant au moins trois zones de soudure à savoir une zone de soudure d'affaiblissement (6) bordée de deux zones de soudures de mélange (5) ; la station de soudure transversale comprenant au moins trois zones de soudure, à savoir une zone de soudures d'affaiblissement (6) bordée de deux zones de soudures de mélange (5).

15. Dispositif de mise en oeuvre du procédé selon la revendication **14** comprenant : une station de soudure longitudinale comprenant au moins un moyen de soudure, chaque moyen comprenant deux rondelles de soudure de mélange (5) entourant une rondelle de soudure d'affaiblissement (6) pouvant être à encoches, de façon à réaliser une soudure d'affaiblissement (6) discontinue, ou bien sans encoche, de façon à réaliser une soudure d'affaiblissement (6) continue ; et une station de soudure transversale comprenant au moins un moyen de soudure, chaque moyen comprenant deux rondelles de soudure de mélange (5) entourant une rondelle de soudure d'affaiblissement (6) pouvant être à encoches, de façon à réaliser une soudure d'affaiblissement (6) discontinue, ou bien sans encoche, de façon à réaliser une soudure d'affaiblissement (6) continue.

## Patentansprüche

1. Thermischer Kühlartikel (1), der aus mindestens zwei Lagen (4), die übereinandergelegt und mit einer Mischschweißnaht verschweißt sind, um ein Pflaster zu definieren, das mindestens drei Pads (2) umfasst, zusammengesetzt ist, wobei die Mischschweißnaht (5) in der Nähe der Spitze jedes Pads (2) verbreitert ist, so dass die Ecke (2) der Pads durch eine vergrößerte Schweißfläche verstärkt wird, wobei jedes Pad (2) mindestens drei Seiten und drei Spitze umfasst, wobei sich die Pads (2) in mindestens zwei Raumrichtungen erstrecken, die eine Ebene definieren, wobei die Pads (2) an mindestens einer ihrer Seiten nebeneinander liegen und miteinander verbunden sind, wobei die Pads (2) eine Kammer (3) definieren, die ein Raum zwischen den beiden Lagen (4) ist, wobei die Kammer mindestens ein Material enthält, das zum Kühlen, Gefrieren oder Einfrieren konfiguriert ist, das aus einem Absorptionsmaterial für wässrige Medien oder einem Füllmaterial ausgewählt ist, wobei die Pads (2) geschlossen und durch mindestens eine Schweißnaht miteinander verbunden sind,
***dadurch gekennzeichnet, dass*** die Schweißnaht aus einer zerbrechlichen Linie (8) besteht, die aus einer regelmäßigen Folge von Schwächungsschweißnähten (6) und Verbindungspunkten (7) besteht, wobei die Schwächungsschweißnaht (6) zwischen zwei Pads (2) im Bereich der Spitze jedes Pads (2) durchgehend ist.

2. Thermischer Kühlartikel (1) nach Anspruch **1**, wobei mindestens eine der Lagen für ein wässriges Medium durchlässig ist und das Material ein Absorptionsmaterial für wässrige Medien ist, das durch Kontakt mit dem wässrigen Medium aktivierbar ist, wobei die Aktivierung dazu führt, dass eine Mischung aus Wasser und Absorptionsmaterial innerhalb der Kammer aufquillt.

3. Thermischer Kühlartikel (1) nach Anspruch **1** oder **2**, wobei das Absorptionsmaterial für wässrige Medien Polyacrylatpulver ist.

4. Thermischer Kühlartikel (1) nach einem der Ansprüche **1** bis **3**, wobei mindestens eine der Lagen (4) und vorzugsweise jede der beiden Lagen (4) aus einer Überlagerung mehrerer Materialstärken besteht.

5. Thermischer Kühlartikel (1) nach einem der Ansprüche **1** bis **4,** wobei die Schwächungsschweißnaht (6) zwischen zwei Pads (2) an mindestens zwei Punkten je Zentimeter derart diskontinuierlich ist, dass mindestens zwei Verbindungspunkte (7) der Mischschweißnaht (5) erzeugt werden.

6. Thermischer Kühlartikel (1) nach einem der Ansprüche **1** bis **5**, wobei die Fläche der Kammer (3) 10 bis 99 %, vorzugsweise 50 bis 95 %, sehr bevorzugt 70 bis 90 % der Fläche des Pads (2) einnimmt.

7. Thermischer Kühlartikel (1) nach einem der Ansprüche **1** bis **6**, wobei mindestens eine der Außenseiten der Lagen (4) ganz oder teilweise mit einem Haftmittel beschichtet ist.

8. System, umfassend eine Verpackung und einen thermischen Kühlartikel (1) nach einem der Ansprüche **1** bis **7**, wobei sich der thermische Kühlartikel (1) in der Verpackung befindet, die mindestens eine mikroporöse oder mikroperforierte Folie umfasst, die wasserdurchlässig, aber eine Barriere für Mikroben, Bakterien und/oder Viren ist.

9. Vorrichtung, umfassend eine mehrteilige Beutelverpackung (10) und einen thermischen Kühlartikel (1) nach einem der Ansprüche **1** bis **7,** wobei mehrteilige Beutelverpackung (10) umfasst:
- einen ersten Beutel (101), der den thermischen Kühlartikel (1) umfasst,
- einen zweiten Beutel (102), der vom ersten Beutel unabhängig und in Bezug auf den ersten Beutel dicht ist und ein wässriges Medium enthält oder aufnehmen kann,
- wobei die beiden Beutel an einer ihrer Seiten fest miteinander verbunden sind, und
- wobei die beiden Beutel ein Mittel (12) zur Herstellung einer Fluidverbindung des zweiten Beutels zum ersten Beutel umfassen.

10. Thermische Kühlkompresse, die mindestens einen thermischen Kühlartikel (1) nach einem der Ansprüche **1** bis **7** umfasst, wobei das Material ein Absorptionsmaterial für wässrige Medien ist, das durch Kontakt mit einem wässrigen Medium aktiviert wurde und das gekühlt, gefroren oder tiefgefroren wurde.

11. Thermische Kühlkompresse, die mindestens einen thermischen Kühlartikel (1) nach einem der Ansprüche **1** bis **7** umfasst, wobei das Material ein Füllmaterial ist, das gekühlt, gefroren oder tiefgefroren wurde.

12. Rolle, die mindestens einen thermischen Kühlartikel (1) nach einem der Ansprüche **1** bis **7** oder mindestens ein System nach Anspruch **8** umfasst, das auf sich selbst gewickelt ist.

13. Helm, vorzugsweise Chemotherapie-Helm zur Bekämpfung von Haarausfall im Zusammenhang mit einer Chemotherapie-Behandlung, umfassend eine Haube, vorzugsweise eine Einweghaube, und mindestens einen thermischen Kühlartikel (1) nach einem der Ansprüche **1** bis **7**, mindestens ein System nach Anspruch **8** oder mindestens eine Vorrichtung nach Anspruch **9.**

14. Verfahren zur kontinuierlichen Herstellung eines thermischen Kühlartikels (1) nach einem der Ansprüche **1** bis **7**, eines Systems nach Anspruch **8** oder einer Vorrichtung nach Anspruch **9**, umfassend einen kontinuierlichen Durchlauf von mindestens zwei nicht verschweißten Lagen (4) an zwei Schweißstationen vorbei: eine Längsschweißstation, eine Querschweißstation; wobei die Längsschweißstation mindestens drei Schweißzonen umfasst, nämlich eine Schwächungsschweißzone (6), die von zwei Mischschweißzonen (5) begrenzt wird; wobei die Querschweißstation mindestens drei Schweißzonen umfasst, nämlich eine Schwächungsschweißzone (6), die von zwei Mischschweißzonen (5) begrenzt wird.

15. Vorrichtung zur Durchführung des Verfahrens nach Anspruch **14**, umfassend: eine Längsschweißstation mit mindestens einem Schweißmittel, wobei jedes Mittel zwei Mischschweißscheiben (5) umfasst, die eine Schwächungsschweißscheibe (6) umgeben, die gekerbt sein kann, um eine diskontinuierliche Schwächungsschweißung (6) zu erzeugen, oder auch nicht gekerbt sein kann, um eine kontinuierliche Schwächungsschweißung (6) zu erzeugen; und eine Querschweißstation mit mindestens einem Schweißmittel, wobei jedes Mittel zwei Mischschweißscheiben (5) umfasst, die eine Schwächungsschweißscheibe (6) umgeben, wobei die Schwächungsschweißnaht gekerbt sein kann, um eine diskontinuierliche Schwächungsschweißnaht (6) zu erzeugen, oder die nicht gekerbt sein kann, um eine kontinuierliche Schwächungsschweißnaht (6) zu erzeugen.

## Claims

1. A cooling thermal article (1) composed of at least two superimposed sheets (4) welded by a mixing weld to define a paving comprising at least three tiles (2), said mixing weld (5) is widened near the apex of each tile (2) so that the corner of the tiles (2) is reinforced by an increased weld surface, each tile (2) comprising at least three sides and three apexes, said tiles (2) extending in at least two directions of space defining a plane, said tiles (2) being adjacent and connected to each other by at least one of their sides, said tiles (2) defining a compartment (3), which is a space between the two sheets (4), the compartment including at least one material configured to be cooled, frozen or deep-frozen, selected from an aqueous medium absorbent material or a filler material, said tiles (2) being closed and connected to each other by at least one weld, **characterized in that** the weld is composed of a frangible line (8) consisting of a regular succession of weakening welds (6) and junction points (7), the weakening weld (6) between two tiles (2) being continuous at the top of each tile (2).

2. A thermal cooling article (1) according to claim 1, wherein at least one of the laminates is permeable to an aqueous medium and the material is an aqueous medium absorbent material which is activatable by contact with the aqueous medium, said activation causing a mixture of water and absorbent material to swell within the compartment.

3. A thermal cooling article (1) according to claim 1 or 2, wherein the aqueous medium absorbent material is polyacrylate powder.

4. Thermal cooling article (1) according to one of claims 1 to 3, in which at least one of the sheets (4) and preferably each of the two sheets (4) consists of a superposition of several thicknesses of material.

5. Thermal cooling article (1) according to any one of claims 1 to 4, wherein the weakening weld (6) between two tiles (2) is discontinuous at at least two points per centimeter, so as to generate at least two junction points (7) of mixing weld (5).

6. Thermal cooling article (1) according to any one of claims 1 to 5, wherein the surface of the compartment (3) occupies 10 to 99%, preferably 50 to 95%, very preferably 70 to 90% of the surface of the tile (2).

7. Thermal cooling article (1) according to any one of claims 1 to 6, wherein at least one of the outer faces of the sheets (4) is coated, wholly or partially, with an adhesive.

8. A system comprising a package and a cooling thermal article (1) according to any one of claims 1 to 7, wherein said cooling thermal article (1) is in said package which comprises at least one microporous or microperforated film permeable to water but a barrier to microbes, bacteria and/or viruses.

9. A device comprising a multi-pocket package (10) and a thermal cooling article (1) according to any one of claims 1 to 7, said multi-pocket package (10) comprising:
- a first pocket (101) comprising said cooling thermal article (1),
- a second pouch (102), independent of the first pouch and sealed with respect to the first pouch, comprising or suitable for receiving an aqueous medium,
- the two pockets being integral on one of their sides, and
- the two pouches comprising means (12) for bringing the second pouch into fluid communication with the first pouch.

10. A cooling thermal compress comprising at least one cooling thermal article (1) according to any one of claims 1 to 7, wherein the material is an aqueous medium absorbent material which has been activated by contact with an aqueous medium, and which has been cooled, frozen or deep-frozen.

11. A cooling thermal compress comprising at least one cooling thermal article (1) according to any one of claims 1 to 7, wherein the material is a filler material which has been cooled, frozen or deep-frozen.

12. A roll comprising at least one thermal cooling article (1) according to any one of claims 1 to 7 or at least one system according to claim 8 wound on itself.

13. A helmet, preferably a chemotherapy helmet for combating hair loss associated with chemotherapy treatment, comprising a charlotte, preferably disposable, and at least one cooling thermal article (1) according to any one of claims 1 to 7, at le Casque, de préférence casque de chimiothérapie pour lutter contre la chute des cheveux liée à un traitement de chimiothérapie, comprenant une charlotte, de préférence jetable, et au moins un article (1) thermique refroidissant selon l'une quelconque des revendications 1 à 7, au moins un système selon la revendication 8 ou au moins un dispositif selon la revendication 9.

14. Process for the continuous manufacture of a cooling thermal article (1) according to any one of claims 1 to 7, of a system according to claim 8 or of a device according to claim 9 comprising a continuous unwinding of at least two non-welded sheets (4) in front of two welding stations: one longitudinal, one transverse; the longitudinal welding station comprising at least three welding zones, namely a weakening welding zone (6) bordered by two mixing welding zones (5); the transverse welding station comprising at least three welding zones, namely a weakening welding zone (6) bordered by two mixing welding zones (5).

15. Device for carrying out the process according to claim 14 comprising : a longitudinal welding station comprising at least one welding means, each means comprising two mixing weld washers (5) surrounding a weakening weld washer (6) which may be notched, so as to produce a discontinuous weakening weld (6), or un-notched, so as to produce a continuous weakening weld (6) ; and a transverse welding station comprising at least one welding means, each means comprising two mixing weld washers (5) surrounding a weakening weld washer (6), which may be notched, so as to produce a discontinuous weakening weld (6), or unnotched, so as to produce a continuous weakening weld (6).
